# EUROPÄISCHE PATENTSCHRIFT

(11) **EP 1 782 763 B1**
(45) Veröffentlichungstag und Bekanntmachung des Hinweises auf die Patenterteilung: **27.02.2008**
(21) Anmeldenummer: 06020668.7
(22) Anmeldetag: 30.09.2006
(51) Int. Cl.: A61F 2/16

(54) **Iris fixierte Intraokularlinse**
Iris fixated intraocular lens
Lentille intraoculaire à fixation iridienne

(30) Priorität: 04.11.2005 DE 102005053078
(43) Veröffentlichungstag der Anmeldung: 09.05.2007
(73) Patentinhaber: Dr. Schmidt Intraocularlinsen GmbH, 53757 St. Augustin (DE)
(72) Erfinder: Meßner, Arthur, 91220 Schnaittach (DE)
(74) Vertreter: Rau, Albrecht

(56) Entgegenhaltungen:
- WO-A-20/05055891
- US-A- 5 192 319
- US-A- 6 152 959

## Beschreibung

Die Erfindung betrifft eine Intraokularlinse zur Implantation in die Vorderkammer eines Auges, insbesondere eines menschlichen Auges.

Intraokularlinsen zur Implantation in die Vorderkammer eines Auges sind seit langem bekannt und werden entweder nach Entfernung der natürlichen Linse als Ersatz für diese oder zusätzlich zu der natürlichen Linse zur Korrektur einer Fehlsichtigkeit in das menschliche Auge implantiert. Intraokularlinsen, die zusätzlich ohne Entfernung der natürlichen Linse implantiert werden, werden auch als phake Intraokularlinsen bezeichnet. Eine derartige Linse ist beispielsweise aus der US 5 192 319 bekannt. Bei der Entwicklung und Anwendung derartiger Intraokularlinsen besteht ein stetiges Bedürfnis, die Verträglichkeit und die Abbildungseigenschaften der implantierten Intraokularlinsen zu verbessern.

Der Erfindung liegt die Aufgabe zugrunde, eine Intraokularlinse zur Implantation in die Vorderkammer eines Auges zu schaffen, bei der postoperative Komplikationen und Abbildungsfehler im Auge vermieden werden.

Diese Aufgabe wird durch die Merkmale des Anspruchs 1 gelöst. Der Kern der Erfindung besteht darin, dass die anteriore und posteriore Optik-Kante als Übergang zwischen den Optik-Flächen und der Rand-Fläche scharfkantig ausgebildet sind und die Rand-Fläche im Wesentlichen entlang der optischen Achse verläuft. Durch die Optik-Kanten und die Rand-Fläche verursachte Abbildungsfehler werden somit im Wesentlichen vermieden. Die Optik-Flächen sind in Folge des scharfkantigen Übergangs zu der Rand-Fläche vollflächig, das heißt auch in deren Randbereich, nutzbar. Mittels der Haptik-Arme ist die Optik einfach und gut verträglich an der Iris des Auges befestigbar.

Weitere vorteilhafte Ausgestaltungen der Erfindung ergeben sich aus den Unteransprüchen.

Zusätzliche Merkmale und Einzelheiten der Erfindung ergeben sich aus der Beschreibung eines Ausführungsbeispiels anhand der Zeichnung. Es zeigen:

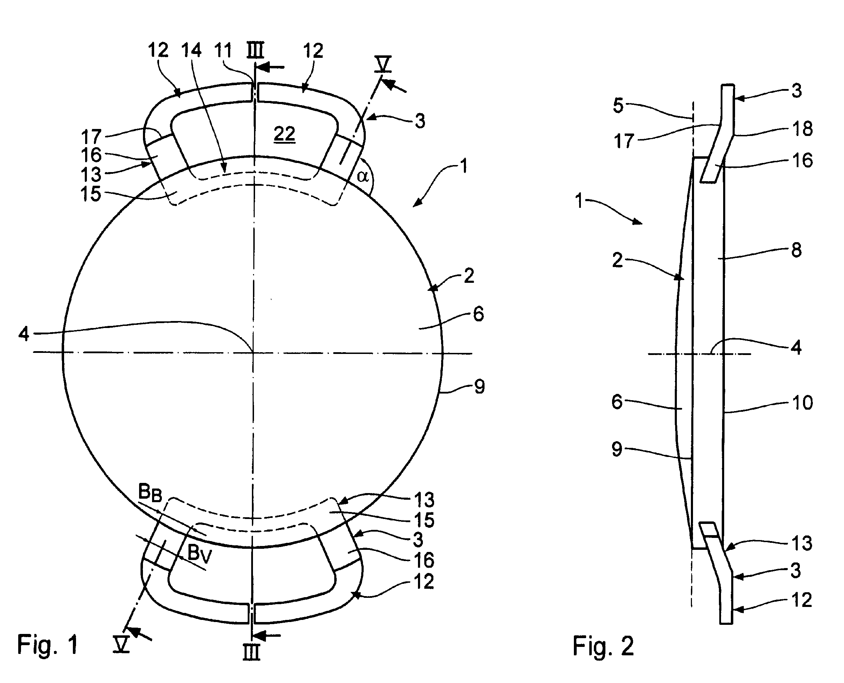

- Fig. 1: eine anteriore Ansicht einer Intraokularlinse,
- Fig. 2: eine seitliche Ansicht der Intraokularlinse in Fig. 1,
- Fig. 3: einen Querschnitt durch die Intraokularlinse entlang der Schnittlinie III-III in Fig. 1,
- Fig. 4: einen vergrößerten Ausschnitt der Intraokularlinse in Fig. 3, und
- Fig. 5: einen Querschnitt durch die Intraokularlinse entlang der Schnittlinie V-V in Fig. 1 mit vereinfachter Darstellung der Haptik-Arme.

Eine Intraokularlinse 1 ist mehrteilig ausgebildet und weist eine zentrale Optik 2 und daran befestigte Haptiken 3 auf. Die Optik 2 besitzt mittig eine optische Achse 4 sowie eine senkrecht an der optischen Achse 4 durch die Optik 2 verlaufende Linsen-Ebene 5.

Die Optik 2 umfasst eine im implantierten Zustand der Hornhaut des Auges zugewandte anteriore Optik-Fläche 6 und eine der anterioren Optik-Fläche 6 relativ zu der Linsen-Ebene 5 gegenüberliegende und der Iris des Auges zugewandte posteriore Optik-Fläche 7. Die Optik-Flächen 6, 7 verlaufen im Wesentlichen quer zu der optischen Achse 4 und sind stetig, das heißt die Optik-Flächen 6, 7 weisen aufgrund des stetigen Verlaufs keine Fresnell-Stufen auf. Vorzugsweise verlaufen die Flächen 6, 7 kontinuierlich gekrümmt. Zwischen den Optik-Flächen 6, 7 erstreckt sich eine konzentrisch und parallel zu der optischen Achse 4 verlaufende, ringförmige Rand-Fläche 8. Die Rand-Fläche 8 wird in Richtung der anterioren Optik-Fläche 6 von einer kreisförmigen anterioren Optik-Kante 9 und in Richtung der posterioren Optik-Fläche 7 von einer kreisförmigen posterioren Optik-Kante 10 begrenzt.

Die anteriore Optik-Kante 9 ist scharfkantig ausgebildet und erstreckt sich entlang eines Teilkreisbogens mit einem Radius R₁ von kleiner als 100 µm, insbesondere von kleiner als 50 µm, und insbesondere von kleiner als 10 µm. Die posteriore Optik-Kante 10 ist ebenfalls scharfkantig ausgebildet und erstreckt sich entlang eines Teilkreisbogens mit einem Radius R₂ von kleiner als 100 µm, insbesondere von kleiner als 50 µm, und insbesondere von kleiner als 10 µm.

Die anteriore Optik-Fläche 6 ist relativ zu der Linsen-Ebene 5 konvex gekrümmt und weist einen Krümmungsradius R_{A} im Bereich von 3 mm bis 90 mm, insbesondere von 4 mm bis 80 mm, und insbesondere von 5 mm bis 70 mm auf. Alternativ kann die anteriore Optik-Fläche 6 relativ zu der Linsen-Ebene 5 auch konkav gekrümmt sein und einen Krümmungsradius R_{A} in den genannten Bereichen aufweisen. Die posteriore Optik-Fläche 7 ist relativ zu der Linsen-Ebene 5 konkav gekrümmt und weist einen Krümmungsradius R_{P} im Bereich von 4 mm bis 20 mm, insbesondere von 6 mm bis 16 mm, und insbesondere von 8 mm bis 12 mm auf.

Die Optik-Flächen 6, 7 sind derart ausgebildet, dass eine Wellenfrontdeformation im Wesentlichen mit Zernike-Polynomen bis zu einer Ordnung 8, insbesondere bis zu einer Ordnung 6, und insbesondere bis zu einer Ordnung 4 mathematisch beschreibbar ist. Zernike-Polynome sind ein qualitatives und quantitatives Maß für die Abbildungsfehler der Optik 2.

Die Optik 2 ist einteilig aus einem Optik-Material hergestellt, wobei als Optik-Material beispielsweise Silikon mit einem Brechungsindex von ungefähr 1,43 dient. Alternativ ist als Optik-Material ein hydrophiles Acrylat mit einem Brechungsindex von 1,46 oder ein hochbrechendes Optik-Material mit einem Brechungsindex von größer als 1,5 einsetzbar.

Die zwei an der Optik 2 befestigten Haptiken 3 erstrecken sich radial nach außen und sind relativ zu der optischen Achse 4 einander gegenüberliegend angeordnet. Alternativ ist es auch möglich eine andere Anzahl von Haptiken vorzusehen, solange diese gleichmäßig über den Umfang der Optik verteilt sind. Die Haptiken 3 sind identisch ausgebildet und an der Optik 2 befestigt, so dass nachfolgend lediglich eine Haptik 3 genau beschrieben ist.

Die einteilig ausgebildete Haptik 3 weist zwei einen Einklemm-Spalt 11 begrenzende Haptik-Arme 12 und einen die Haptik-Arme 12 verbindenden Haptik-Bügel 13 auf. Der Haptik-Bügel 13 umfasst einen in einer Optik-Ausnehmung 14 aufgenommenen und von dem Optik-Material vollständig umgebenen Befestigungs-Abschnitt 15 und daran angeordnete, freiliegende Verbindungs-Abschnitte 16. Der Befestigungs-Abschnitt 15 verläuft mittig konzentrisch zu der optischen Achse 4 und ist endseitig derart abgewinkelt, dass er benachbart zu den Verbindungs-Abschnitten 16 und entsprechend zu diesen radial nach außen verläuft. Der Haptik-Bügel 13 durchbricht die Rand-Fläche 8 - umfänglich betrachtet - mit einem Winkel α von ungefähr 90°. Der Haptik-Bügel 13 verläuft im Wesentlichen tangential und bündig zu der posterioren Optik-Fläche 7 und erstreckt sich bis zu einer anterioren Haptik-Kante 17 und einer posterioren Haptik-Kantet 18. Die Verbindungs-Abschnitte 16 schließen - entlang der optischen Achse 5 betrachtet - mit der Rand-Fläche 8 einen dem Krümmungsradius R_{P} der posterioren Optik-Fläche 7 angepassten Winkel β ein. Die anteriore Haptik-Kante 17 und die posteriore Haptik-Kante 18 verlaufen im Wesentlichen parallel zu der Rand-Fläche 8, wobei die anteriore Haptik-Kante 17 aufgrund der Schrägstellung des Haptik-Bügels 13 relativ zu der Linsen-Ebene 5 einen größeren Abstand zu der optischen Achse 4 als die posteriore Haptik-Kante 18 aufweist.

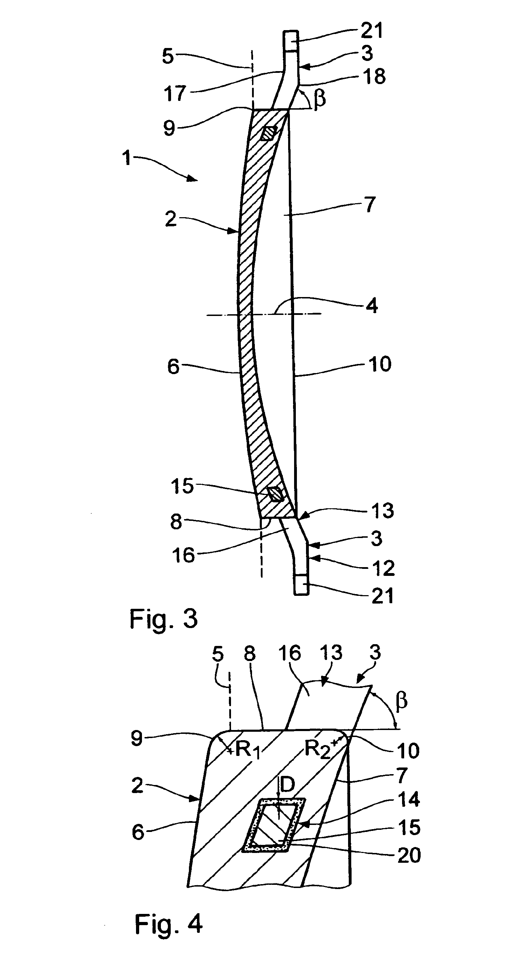

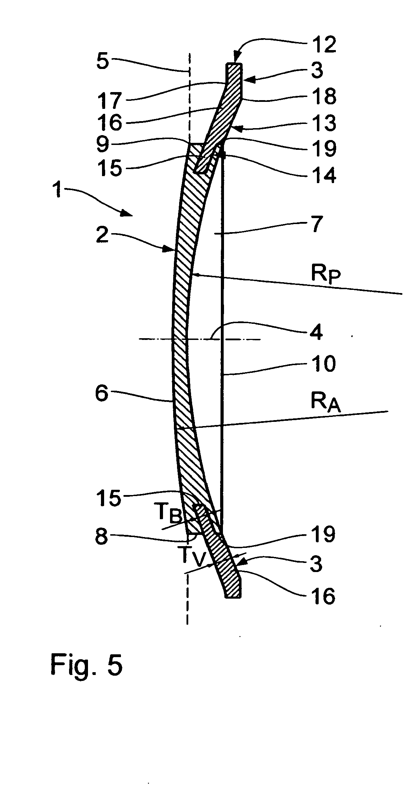

Der Befestigungs-Abschnitt 15 weist mittig einen Querschnitt in Form eines Parallelogramms auf, wobei eine obere und eine untere Seitenwand parallel zu der Rand-Fläche 8 verlaufen, wie die Fig. 3 und 4 zeigen. Im Bereich der Rand-Fläche 8 vergrößert sich der Querschnitt des Befestigungs-Abschnitts 15 derart, dass entlang der Rand-Fläche 8 eine Breite B_{B} des Befestigungs-Abschnitts 15 einer Breite B_{V} der Verbindungs-Abschnitte 16 entspricht. Entlang der optischen Achse 4 betrachtet weisen die Verbindungs-Abschnitte 16 eine Tiefe T_{V} auf, die im Vergleich zu einer Tiefe T_{B} des Befestigungs-Abschnitts 15 größer ist, so dass die Verbindungs-Abschnitte 16 einen sich in Richtung der posterioren Optik-Kante 10 erstreckenden Vorsprung 19 ausbilden, der an der Rand-Fläche 8 anliegt, wie Fig. 5 zeigt.

Zwischen dem Befestigungs-Abschnitt 15 des Haptik-Bügels 13 und dem Optik-Material ist eine Beschichtung 20 angeordnet, die für Licht des sichtbaren Spektrums undurchlässig ist. Die Beschichtung 20 weist eine Dicke D von mindestens 0,5 µm, insbesondere von mindestens 1 µm, und insbesondere von mindestens 5 µm auf. Alternativ zu der Beschichtung kann die Oberfläche des Befestigungs-Abschnitts 15 aufgeraut sein.

Ausgehend von den anterioren Haptik-Kanten 17 und den posterioren Haptik-Kanten 18 erstrecken sich die Haptik-Arme 12 parallel zu der Linsen-Ebene 5. Die Haptik-Arme 12 verlaufen ausgehend von den Haptik-Kanten 17, 18 zunächst radial nach außen und biegen sich im Weiteren derart, dass sie im Wesentlichen parallel zu der Rand-Fläche 8 und aufeinander zu verlaufen. An ihren freien Enden weisen die Haptik-Arme 12 senkrecht zu der Linsen-Ebene 5 verlaufende Stirnflächen 21 auf, die den Einklemm-Spalt 11 seitlich begrenzen.

Zur Befestigung der Intraokularlinse 1 an der Iris weisen die Haptiken 3 an den Stirnflächen 21 eine mittlere Rautiefe von mindestens 5 µm, insbesondere von mindestens 10 µm, und insbesondere von mindestens 20 µm auf. Die mittlere Rautiefe der weiteren Haptik-Flächen beträgt weniger als 4 µm, insbesondere weniger als 3 µm, und insbesondere weniger als 2,5 µm.

Die Rand-Fläche 8 der Optik 2, die Verbindungs-Abschnitte 16 des Haptik-Bügels 13 und die Haptik-Arme 12 begrenzen im Wesentlichen eine Haptik-Ausnehmung 22. Die Haptik-Ausnehmung 22 steht mit dem Einklemm-Spalt 11 in Verbindung.

Die Haptiken 3 sind einteilig aus einem Haptik-Material geformt, wobei als Haptik-Material insbesondere PMMA dient. Das Haptik-Material weist ein erstes Elastizitätsmodul E_{H} und das Optik-Material ein zweites Elastizitätsmodul E_{O} auf, wobei vorzugsweise das Verhältnis der Elastizitätsmodule E_{H}/E_{O} größer als 1,5, insbesondere größer als 2, und insbesondere größer als 3 ist. Ferner weist das Haptik-Material einen ersten Brechungsindex B_{H} und das Optik-Material einen zweiten Brechungsindex B_{O} auf, wobei die Differenz der Brechungsindizes B_{H} - B_{O} betragsmäßig mindestens 0,03, insbesondere 0,06 und insbesondere 0,09 ist.

Die Intraokularlinse 1 wird in die Vorderkammer eines Auges implantiert. Sie dient entweder als Ersatz für die natürliche Linse, die beispielsweise aufgrund von Grauem Star entfernt wurde, oder als Zusatz zu der natürlichen Linse um eine Fehlsichtigkeit zu korrigieren. Die Intraokularlinse 1 wird durch einen Schnitt in der Hornhaut in die Vorderkammer des Auges eingeführt und mittels der Haptik-Arme 12 an der Iris befestigt. Hierzu wird die Iris zwischen den Haptik-Armen 12 in den Einklemm-Spalt 11 der Haptiken 3 eingeklemmt. Die Breite des Einklemm-Spalts 11 und die mittlere Rautiefe der Stirnflächen 21 ist derart bemessen, dass die Intraokularlinse 1 mit den Haptiken 3 sicher an der Iris befestigt ist. Die Haptik-Arme 12 liegen mit ihren der Iris zugewandten, posterioren Seitenflächen an der Iris an, so dass die Optik 2 aufgrund der schräg zu der Linsen-Ebene 5 verlaufenden Haptik-Bügeln 13 von der Iris abgehoben ist und frei liegt. Dadurch, dass lediglich die Haptik-Arme 12 mit der Iris in Kontakt sind, weist die Intraokularlinse 1 eine gute Verträglichkeit auf. Die scharfkantige Ausbildung der Optik-Kanten 9, 10 und die parallel zu der optischen Achse 4 verlaufende Rand-Fläche 8 reduzieren Abbildungsfehler, so dass die Intraokularlinse 1 sehr gute Abbildungseigenschaften aufweist. Die Geometrie der Haptiken 3 und deren Befestigung an der Optik 2 ermöglichen eine gleichmäßige Krafteinleitung in die Optik 2 bei auf die Haptiken 3 wirkenden Kräften wodurch Abbildungsfehler aufgrund einer Deformation der Optik 2 reduziert werden. Zusätzlich reduziert die Beschichtung 20 zwischen dem Haptik-Material und dem Optik-Material Abbildungsfehler durch störende Lichteffekte aufgrund der in dem Optik-Material verlaufenden Haptik-Bügel 13.

## Patentansprüche

1. Intraokularlinse zur Implantation in ein Auge, mit
a. einer aus einem Optik-Material bestehenden Optik (2), die
i. eine optische Achse (4),
ii. eine quer zu der optischen Achse (4) und stetig verlaufende anteriore Optik-Fläche (6),
iii. eine der anterioren Optik-Fläche (6) gegenüberliegende und stetig verlaufende posteriore Optik-Fläche (7),
iv. eine zwischen den Optik-Flächen (6, 7) und entlang der optischen Achse (4) verlaufende Rand-Fläche (8),
v. eine die Rand-Fläche (8) und die anteriore Optik-Fläche (6) begrenzende und scharfkantig ausgebildete anteriore Optik-Kante (9), und
vi. eine die Rand-Fläche (8) und die posteriore Optik-Fläche (7) begrenzende und scharfkantig ausgebildete posteriore Optik-Kante (10) aufweist, und
b. mindestens zwei aus einem Haptik-Material bestehenden und an der Optik (2) befestigten Haptiken (3), die zur Befestigung der Intraokularlinse (1) an der Iris jeweils mindestens zwei Haptik-Arme (12) aufweisen,
c. **dadurch gekennzeichnet, dass** das Haptik-Material ein erstes Elastizitätsmodul E_{H} und das Optik-Material ein zweites Elastizitätsmodul E_{O} aufweist, wobei das Verhältnis der Elastizitätsmodule E_{H}/E_{O} größer als 1,5 ist.

2. Intraokularlinse nach Anspruch 1, **dadurch gekennzeichnet, dass** sich die anteriore Optik-Kante (9) entlang eines Teilkreisbogens mit einem Radius (R₁) von kleiner als 100 µm, insbesondere von kleiner als 50 µm, und insbesondere von kleiner als 10 µm erstreckt.

3. Intraokularlinse nach Anspruch 1 oder 2, **dadurch gekennzeichnet, dass** sich die posteriore Optik-Kante (10) entlang eines Teilkreisbogens mit einem Radius (R₂) von kleiner als 100 µm, insbesondere von kleiner als 50 µm, und insbesondere von kleiner als 10 µm erstreckt.

4. Intraokularlinse nach einem der Ansprüche 1 bis 3, **dadurch gekennzeichnet, dass** die Optik-Flächen (6, 7) derart ausgebildet sind, dass eine durch die Intraokularlinse hervorgerufene Wellenfrontdeformation im Wesentlichen mit Zernike-Polynomen bis zu einer Ordnung 8, insbesondere bis zu einer Ordnung 6, und insbesondere bis zu einer Ordnung 4 beschreibbar ist.

5. Intraokularlinse nach einem der Ansprüche 1 bis 4, **dadurch gekennzeichnet, dass** zur Befestigung der mindestens zwei Haptik-Arme (12) an der Optik (2) jeweils ein zumindest teilweise von dem Optik-Material umschlossener Haptik-Bügel (13) vorgesehen ist.

6. Intraokularlinse nach Anspruch 5, **dadurch gekennzeichnet, dass** zwischen dem Haptik-Bügel (13) und dem Optik-Material eine für Licht des sichtbaren Spektrums undurchlässige Beschichtung (20) vorgesehen ist.

7. Intraokularlinse nach Anspruch 6, **dadurch gekennzeichnet, dass** die Beschichtung (20) eine Dicke (D) von mindestens 0,5 µm, insbesondere von mindestens 1 µm, und insbesondere von mindestens 5 µm aufweist.

8. Intraokularlinse nach einem der Ansprüche 1 bis 7, **dadurch gekennzeichnet, dass** die anteriore Optik-Fläche (6) relativ zu einer Linsen-Ebene (5) konvex oder konkav gekrümmt ist und einen Krümmungsradius (R_{A}) im Bereich von 3 mm bis 90 mm, insbesondere von 4 mm bis 80 mm, und insbesondere von 5 mm bis 70 mm aufweist.

9. Intraokularlinse nach einem der Ansprüche 1 bis 7, **dadurch gekennzeichnet, dass** die posteriore Optik-Fläche (7) relativ zu einer Linsen-Ebene (5) konkav gekrümmt ist und einen Krümmungsradius (R_{P}) im Bereich von 4 mm bis 20 mm, insbesondere von 6 mm bis 16 mm, und insbesondere von 8 mm bis 12 mm aufweist.

## Claims

1. Intraocular lens for implantation in an eye, with
a. an optic (2) which consists of an optic material and has
i. an optical axis (4),
ii. an anterior optic surface (6) extending continuously and transversely to the optical axis (4),
iii. a posterior optic surface (7) extending continuously and opposite the anterior optic surface (6),
iv. a peripheral surface (8) extending between the optic surfaces (6, 7) and along the optical axis (4),
v. a sharp-edged anterior optic edge (9) defining the peripheral surface (8) and the anterior optic surface (6), and
vi. a sharp-edged posterior optic edge (10) defining the peripheral surface (8) and the posterior optic surface (7), and
b. at least two haptics (3) which are attached to the optic (2), consist of a haptic material and have in each case two haptic arms (12) for attaching the intraocular lens (1) to the iris,
c. **characterised in that** the haptic material has a first modulus of elasticity (E_{H}) and the optic material has a second modulus of elasticity (E_{O}), the ratio of the modules of elasticity (E_{H}/E_{O}) being greater than 1.5

2. Intraocular lens according to claim 1, **characterised in that** the anterior optic edge (9) extends along a partial arc of a circle with a radius (R₁) of less than 100 µm, particularly less than 50 µm and in particular less than 10 µm.

3. Intraocular lens according to claim 1 or 2, **characterised in that** the posterior optic edge (10) extends along a partial arc of a circle with a radius (R₂) of less than 100 µm, particularly less than 50 µm and in particular less than 10 µm.

4. Intraocular lens according to any one of claims 1 to 3, **characterised in that** the optic surfaces (6, 7) are configured in such a way that it is possible to describe a wavefront deformation, caused by the intraocular lens, substantially with Zernike polynomials up to an order of 8, particularly up to an order of 6, and in particular up to an order of 4.

5. Intraocular lens according to any one of claims 1 to 4, **characterised in that** a haptic strap (13) at least partly enclosed by the optic material is provided in each case to attach the at least two haptic arms (12) to the optic (2).

6. Intraocular lens according to claim 5, **characterised in that** a coating (20) impermeable to light of the visible spectrum is provided between the haptic strap (13) and the optic material.

7. Intraocular lens according to claim 6, **characterised in that** the coating (20) has a thickness (D) of at least 0.5 µm, particularly at least 1 µm, and in particular at least 5 µm.

8. Intraocular lens according to any one of claims 1 to 7, **characterised in that** the anterior optic surface (6) is curved in convex or concave manner relative to a lens plane (5) and has a radius of curvature (R_{A}) ranging from 3 mm to 90 mm, particularly from 4 mm to 80 mm, and in particular from 5 mm to 70 mm.

9. Intraocular lens according to any one of claims 1 to 7, **characterised in that** the posterior optic surface (7) is curved in concave manner relative to a lens plane (5) and has a radius of curvature (R_{P}) ranging from 4 mm to 20 mm, particularly from 6 mm to 16 mm and in particular from 8 mm to 12 mm.

## Revendications

1. Lentille intraoculaire pour implantation dans un oeil, avec
a. une optique (2) constituée d'un matériau optique, qui présente
i. un axe optique (4)
ii. une surface d'optique antérieure (6) orientée en continue et à la transversale de l'axe optique (4)
iii. une face d'optique postérieure (7) opposée à la face d'optique antérieure (6) et continue
iv. une bordure (8) allant entre les faces d'optique (6, 7) et le long de l'axe optique (4),
v. un angle d'optique antérieur (9) angulaire délimitant la bordure (8) et la face d'optique antérieure (6), et
vi. un angle d'optique postérieur (10) angulaire, délimitant la bordure (8) et la face d'optique postérieure (7) et
b. au moins deux haptiques (3) constituées d'un matériau haptique et fixées à l'optique (2), qui présentent chacune au moins deux pattes haptiques (12) pour la fixation de la lentille oculaire (1) sur l'iris,
c. **caractérisée en ce que** le matériau d'haptique présente un premier module d'élasticité E_{H} et le matériau d'optique un module d'élasticité E_{O}, où le rapport des modules d'élasticité E_{H}/E_{O} est supérieur à 1,5.

2. Lentille intraoculaire selon la revendication 1, **caractérisée en ce que** le pourtour de l'optique antérieur (9) s'étend le long d'une portion de sphère, avec un rayon (R₁) de moins de 100 µm, en particulier de moins de 50 µm et tout particulièrement de moins de 10 µm.

3. Lentille intraoculaire selon la revendication 1 ou 2, **caractérisée en ce que** le pourtour de l'optique postérieur (10) s'étend le long d'une portion de sphère, avec un rayon (R₂) de moins de 100 µm, en particulier de moins de 50 µm et tout particulièrement de moins de 10 µm.

4. Lentille intraoculaire selon l'une des revendications 1 à 3, **caractérisée en ce que** les faces d'optique (6, 7) sont conçues de telles sorte qu'une déformation du front d'ondes, produite par la lentille intraoculaire, peut être décrite essentiellement avec des polynômes de Zernike comme jusqu'à un ordre 8, en particulier jusqu'à un ordre 6, et tout particulièrement jusqu'à un ordre 4.

5. Lentille intraoculaire selon l'une des revendications 1 à 4, **caractérisée en ce que** pour la fixation de chaque patte haptique (12), au minimum de deux, sur l'optique (2), est prévue une bride d'haptique (13), entourée, du moins partiellement, par le matériau d'optique.

6. Lentille intraoculaire selon la revendication 5, **caractérisée en ce qu'**entre la bride d'haptique (13) et le matériau d'optique, est prévue une couche (20) opaque à la lumière du spectre visible.

7. Lentille intraoculaire selon la revendication 6, **caractérisée en ce que** la couche (20) présente une épaisseur (E) d'au moins 0,5 µm, en particulier d'au moins 1 µm, et tout particulièrement d'au moins 5 µm.

8. Lentille intraoculaire selon l'une des revendications 1 à 7, **caractérisée en ce que** la face d'optique antérieure (6), relativement à un plan de lentille (5), est convexe ou concave et présente un rayon de courbure (R**_{A}**) de l'ordre de 3 mm à 90 mm, en particulier de 4 mm à 80 mm, et tout particulièrement de 5 mm à 70 mm.

9. Lentille intraoculaire selon l'une des revendications 1 à 7, **caractérisée en ce que** la face d'optique postérieure (7), relativement à un plan de lentille (5), est concave et présente un rayon de courbure (R_{P}) de l'ordre de 4 mm à 20 mm, en particulier de 6 mm à 16 mm, et tout particulièrement de 8 mm à 12 mm.
